Europäisches Patentamt

European Patent Office

Office européen des brevets

(11) Veröffentlichungsnummer: **0 297 203 B1**

# (12) EUROPÄISCHE PATENTSCHRIFT

(45) Veröffentlichungstag der Patentschrift: **15.01.92**

(21) Anmeldenummer: **88101531.7**

(22) Anmeldetag: **03.02.88**

Die Akte enthält technische Angaben, die nach
dem Eingang der Anmeldung eingereicht wurden
und die nicht in dieser Patentschrift enthalten sind.

(51) Int. Cl.⁵: **A61K 6/00**, C04B 41/49

(54) **Silanisierungsmittel und Verfahren zur Silanisierung.**

(30) Priorität: **25.05.87 DD 303097**

(43) Veröffentlichungstag der Anmeldung:
**04.01.89 Patentblatt 89/01**

(45) Bekanntmachung des Hinweises auf die
Patenterteilung:
**15.01.92 Patentblatt 92/03**

(84) Benannte Vertragsstaaten:
**AT CH DE FR IT LI SE**

(56) Entgegenhaltungen:
**EP-A- 0 095 587**
**DE-A- 2 757 127**
**DE-A- 3 019 539**
**US-A- 3 423 829**

(73) Patentinhaber: **Heraeus Kulzer GmbH**
**Heraeusstr. 12 - 14**
**W-6450 Hanau(DE)**

(72) Erfinder: **Tiller, Hans-Jürgen, Dr.**
**Prof.-Ibrahim-Strasse 23**
**O-6900 Jena(DE)**
Erfinder: **Schmidt, Albert**
**Heuchelheimer Strasse 139a**
**W-6380 Bad Homburg v.d. H.(DE)**
Erfinder: **Garschke, Adelheid**
**Dr. Wilhelm-Kulz-Strasse 12**
**O-6800 Saalfeld(DE)**
Erfinder: **Schaefer, Roland, Dr.**
**Merianweg 5**
**W-6382 Friedrichsdorf(DE)**

(74) Vertreter: **Grimm, Ekkehard**
**Heraeus Holding GmbH Heraeusstrasse 12 -**
**14**
**W-6450 Hanau/Main(DE)**

## Beschreibung

Anwendungsgebiet der Erfindung

Die Erfindung betrifft ein γ-Methacryloyloxypropyltrimethoxysilan enthaltendes Silanisierungsmittel für die Reparatur von keramischem Zahnersatz mit Kunststoff.

Charakteristik des bekannten Standes der Technik

Schon seit einer Reihe von Jahren werden sowohl in der konservierenden Zahnheilkunde als auch in der Zahnprothetik Silane bzw. Silanisierungsverfahren angewandt, um dentalkeramisches Material und Dentalkunststoff fest miteinander zu verbinden.

So ist es aus DE-OS 30 19 539 bekannt, künstliche Mineralzähne mit silangekoppelten Kunststoff-Haftteilen zu versehen, damit eine chemische Bindung zwischen den künstlichen Zähnen und den aus Kunststoff bestehenden Zahnprothesenplatten geschaffen werden kann. Die Silan-Lösung zur Behandlung der Mineralzähne vor dem Anbringen des Kunststoff-Haftteils besteht dabei vorzugsweise aus γ-Aminopropyltriäthoxysilan, γ-Glycidyloxypropyltrimethoxysilan oder γ-Methacryloyloxypropyltrimethoxysilan in essigsaurem Medium.

In Zahn-, Mund- u. Kieferheilkd. 72, 1984, 223 - 229, wird über die Brauchbarkeit einiger Silane an Stelle des γ-Methacryloyloxypropyltrimethoxysilans als Haftvermittler zwischen dentalkeramischen Massen und Dentalkunststoffen berichtet und auf die ungünstigen Ergebnisse der Reparatur von fraktionierten Metallkeramikverkleidungen mit Silanen als Haftvermittler unter Mundbedingungen hingewiesen.

In DE-OS 35 22 737 wird vorgeschlagen, beschädigte Zähne unter Verwendung von Keramik-Teilen wiederherzustellen. Dazu werden die Keramik-Teile zur Erzeugung einer Silan-Klebemittelschicht mit einer Silan-Lösung (γ-Methacryloyloxypropyltrimethoxysilan und/oder γ-Aminopropyltriäthoxysilan) vorbehandelt, getrocknet und dann über eine kunststoffhaltige Schicht, vorzugsweise aus einem aromatisches Dimethacrylat und anorganischen Füllstoff enthaltenden Composite, mit den Zahnflächen verbunden.

Schäden an festsitzendem keramischen Zahnersatz, zum Beispiel an mit Keramik verblendeten metallischen Kronen und Brücken, lassen sich, ohne daß der Zahnersatz entfernt zu werden braucht, mit polymerisierbaren Dentalmaterialien, die neben den Monomeren, meist Methacrylsäureester, feinteilige anorganische Füllstoffe und Pigmente enthalten und als Composites bezeichnet werden, in ästhetisch zufriedenstellender Weise ausbessern. Silanhaltige Haft- oder Klebemittel, im folgenden als Silanisierungsmittel bezeichnet, die auf die beschädigte Keramik aufgetragen werden, sollen eine möglichst feste und dauerhafte Verbindung zwischen Keramik und Kunststoff schaffen.

Bekannte Silanisierungsmittel dieser Art bestehen zum Beispiel aus Lösungen von 0,5 - 25 Gewichts-% γ-Methacryloyloxypropyltrimethoxysilan und 0,25 - 12 Gewichts-% γ-Glycidoxypropyltrimethoxysilan in Butanol (DE-OS 2 757 127) oder aus anderen Organosilicium-Verbindungen, wie Aminoalkyltrialkoxysilanen (DE-OS 36 10 808).

Sie werden nach dem Reinigen der beschädigten Stellen, Beseitigen aller geschwächten Teile in der Umgebung der beschädigten Stellen, zum Beispiel unter Benutzung eines mit hoher Drehzahl umlaufenden Diamantwerkzeugs, Aufbringen eines Grundiermittels und Trocknen aufgetragen oder nach Ätzen der Keramik-Oberfläche, zum Beispiel mit Fluorwasserstoffsäure, aufgebürstet.

Es hat sich aber gezeigt, daß die sonst sehr wirksamen Silanisierungsmittel, unter Mundbedingungen und auf die als Folge der Beschädigung notwendigerweise mechanisch, zum Beispiel durch Schleifen, bearbeiteten Keramik-Bereiche aufgetragen, weniger zuverlässig sind.

Dadurch wird die Keramik-Oberfläche so geglättet, daß die oberflächliche Zone, in der die Keramik nicht mehr im ursprünglich festen Verbund, sondern in Form kleiner Bruchstücke vorliegt, eine Dicke von weniger als 1 Mikrometer besitzt. Mit der mechanischen Bearbeitung wird gleichzeitig eine chemische Aktivierung der Keramik-Oberfläche erreicht, die die Reaktion mit dem Silanisierungsmittel begünstigt. Es wurde gefunden, daß mit einer solchermaßen bearbeiteten und aktivierten Oberfläche mit dem erfindungsgemäßen Silanisierungsmittel eine bessere Haftung als mit bisher beschriebenen Silanisierungsmitteln erreicht wird.

Daß die Silanisierung mit nichthydrolysiertem Silan so wirksam ist, muß als überraschend angesehen werden. Es wird vermutet, daß der trockene Zustand das Eindringen des Silans in die poröse Keramik-Oberfläche begünstigt und damit die Verankerung des Reparaturmaterials in der Keramik-Oberfläche verbessert wird.

Für das Aufbringen des Silanisierungsmittels gibt es mehrere Möglichkeiten. Es kann zum Beispiel tropfenweise in der letzten Phase des Schleifens oder nach dem mechanischen Reinigen der beschliffenen Oberfläche mit einer Bürste aufgebracht werden. Im letzten Fall kann es vorteilhaft sein, das Silanisierungsmittel schon während der mechanischen Reinigung, indem eine mit dem Silanisierungsmittel befeuchtete Bürste verwendet wird, aufzubringen.

Die Keramik-Oberfläche wird nach dem Aufbringen des Silanisierungsmittels getrocknet und dann mit dem Reparaturmaterial versehen.

Als Reparaturmaterial eignen sich besonders dentale Composites, die durch Kaltpolymerisation und/oder Photopolymerisation zu härten sind (siehe zum Beispiel DE-OS 35 22 737 und EP 95 587).

Es stellt sich daher die Aufgabe, ein Silanisierungsmittel und ein Verfahren zur Silanisierung unter Verwendung dieses Mittels zu finden, um auch unter den oben genannten Bedingungen Keramik und Kunststoff dicht, dauerhaft und spaltfrei miteinander verbinden zu können.

Das die Lösung der Aufgabe darstellende Silanisierungsmittel ist dadurch gekennzeichnet, daß es aus 50 - 95 Volumen-% $\gamma$-Methacryloyloxypropyltrimethoxysilan und 5 - 50 Volumen-% Trimethyläthoxysilan besteht.

Vorzugsweise besteht das Silanisierungsmittel aus 85 - 95 Volumen-% $\gamma$-Methacryloyloxypropyltrimethoxysilan und 5 - 15 Volumen-% Trimethyläthoxysilan.

Besonders bewährt hat sich das Silanisierungsmittel aus 90 Volumen-% $\gamma$-Methacryloyloxypropyltrimethoxysilan und 10 Volumen- % Trimethyläthoxysilan.

Vor dem Aufbringen des Silanisierungsmittels wird der gereinigte keramische Zahnersatz in dem zu reparierenden Bereich mit einem Feinschleifer und einer Drehzahl von 5000 - 25000 Umdrehungen/Minute trocken mechanisch bearbeitet.

Sehr naturgetreu lassen sich die Reparaturen mit anorganische Füllstoffe enthaltendem Kronen- und Brückenmaterial auf Kunststoff-Basis, das in Form von Hals-, Dentin- und Schmelzmasse und häufig auch in mit der von dentalkeramischen Massen übereinstimmender Farbe erhältlich ist, ausführen.

Eine besonders dichte und dauerhafte Bindung zwischen Keramik und Kunststoff bildet sich aus, wenn die silanisierte Keramik-Oberfläche nach dem Trocknen und vor dem Auftragen des relativ hochviskosen Composites mit einem weniger viskosen, zum Beispiel einem Methylmethacrylat enthaltenden oder einem füllstoff-freien, polymerisierbaren Dentalmaterial, imprägniert wird.

Ein auf diese Weise hergestellter Keramik/Kunststoff-Verbund war auch nach sechswöchiger Lagerung in Wasser (37 ° C) völlig frei von Spalten.

Falls bei beschädigtem Zahnersatz aus Metall/Keramik Teile des Metallgerüstes freiliegen, ist es erforderlich, vor der eigentlichen Reparatur das sichtbare Metall mit einem sogenannten Opaker oder Abdeckmittel, wie zum Beispiel aus DE-OS 27 57 127 bekannt, abzudecken.

Ausführungsbeispiel

In der Keramik-Verblendung eines Prüfkörpers aus mit Dentalkeramik verblendetem Metallgerüst wird ein Bruch erzeugt. Die Bruchstelle wird mit einem Feinschleifer (8000 Umdrehungen/Minute) trocken und druckarm beschliffen und mit einer Bürste mechanisch gereinigt. Anschließend wird das Silanisierungsmittel aus 90 Volumen-% $\gamma$-Methacryloyloxypropyltrimethoxysilan und 10 Volumen-% Trimethyläthoxysilan mit dem Pinsel aufgetragen und 2 Minuten lang an der Luft trocknen gelassen. Dann wird die Bruchstelle mit einem handelsüblichen Composite beschichtet und das Composite ausgehärtet.

Bei auf diese Weise und unter Verwendung des durch Bestrahlung mit Licht auszuhärtenden Composites Estilux (Kulzer & Co. GmbH) hergestellten Prüfkörpern wurden um das Vierfache höhere Abscherwerte als bei entsprechend, jedoch unter Verwendung herkömmlicher Silanisierungsmittel hergestellten Prüfkörpern gemessen.

**Patentansprüche**

1. $\gamma$-Methacryloyloxypropyltrimethoxysilan enthaltendes Silanisierungsmittel für die Reparatur von keramischem Zahnersatz mit Kunststoff, dadurch gekennzeichnet, daß es aus 50 - 95 Volumen-% $\gamma$-Methacryloyloxypropyltrimethoxysilan und 5 - 50 Volumen-% Trimethyläthoxysilan besteht.

2. Silanisierungsmittel nach Anspruch 1, dadurch gekennzeichnet, daß es aus 85 - 95 Volumen-% $\gamma$-Methacryloyloxypropyltrimethoxysilan und 5 - 15 Volumen-% Trimethyläthoxysilan besteht.

3. Silanisierungsmittel nach Anspruch 1 oder 2, dadurch gekennzeichnet, daß es aus 90 Volumen-% $\gamma$-Methacryloyloxypropyltrimethoxysilan und 10 Volumen-% Trimethyläthoxysilan besteht.

4. Verfahren zum Silanisieren von mit Kunststoff zu reparierendem keramischem Zahnersatz, dadurch gekennzeichnet, daß der keramische Zahnersatz in dem zu reparierenden Bereich mit einem Feinschleifer und einer Drehzahl von 5000 bis 25000 Umdrehungen/Minute trocken beschliffen und ein Silanisierungsmittel aus 50 - 95 Volumen-% $\gamma$-Methacryloyloxypropyltrimethoxysilan und 5 - 50 Volumen-% Trimethyläthoxysilan auf die beschliffene Oberfläche aufgebracht wird.

5. Verfahren nach Anspruch 4, dadurch gekennzeichnet, daß ein Silanisierungsmittel aus 85 - 95 Volumen-% $\gamma$-Methacryloyloxypropyltrime-

thoxysilan und 5 - 15 Volumen-% Trimethyläthoxysilan aufgebracht wird.

6. Verfahren nach Anspruch 4 oder 5, dadurch gekennzeichnet, daß ein Silanisierungsmittel aus 90 Volumen-% $\gamma$-Methacryloyloxypropyltrimethoxysilan und 10 Volumen-% Trimethyläthoxysilan aufgebracht wird.

7. Verfahren nach Anspruch 4, 5 oder 6, dadurch gekennzeichnet, daß das Silanisierungsmittel während des Schleifens aufgebracht wird.

8. Verfahren nach Anspruch 4, 5 oder 6, dadurch gekennzeichnet, daß die beschliffene Oberfläche mit einer Bürste mechanisch gereinigt wird.

9. Verfahren nach Anspruch 8, dadurch gekennzeichnet, daß mit der Bürste gleichzeitig das Silanisierungsmittel aufgetragen wird.

**Claims**

1. $\gamma$-Methacryloyloxypropyltrimethoxy silane - containing silanising agent for repairing ceramic dentures with plastics characterised in that it consists of 50 to 95 volume % of $\gamma$-methacryloyloxypropyltrimethoxy silane and 5 to 50 volume % of trimethylethoxy silane.

2. A silanising agent according to claim 1, characterised in that it consists of 85 to 95 volume % of $\gamma$-methacryloyloxypropyltrimethoxy silane and 5 to 15 volume % of trimethylethoxy silane.

3. A silanising agent according to claim 1 or claim 2, characterised in that it consists of 90 volume % of $\gamma$-methacryloyloxypropyltrimethoxy silane and 10 volume % of trimethylethoxy silane.

4. A method of silanising ceramic dentures which are to be repaired with plastics materials, characterised in that the ceramic denture is ground in the dry state in the area to be repaired by means of a fine grinder and at a rotational speed of 5,000 to 25,000 r.p.m. and that a silanising agent consisting of 50 to 95 volume % of $\gamma$-methacryloyloxypropyltrimethoxy silane and 5 to 50 volume % of trimethylethoxy silane is applied on the ground surface.

5. A method according to claim 4, characterised in that a silanising agent consisting of 85 to 95 volume % of $\gamma$-

methacryloyloxypropyltrimethoxy silane and 5 to 15 volume % of trimethylethoxy silane is applied.

6. A method according to claim 4 or claim 5, characterised in that a silanising agent consisting of 90 volume % of $\gamma$-methacryloyloxypropyltrimethoxy silane and 10 volume % of trimethylethoxy silane is applied.

7. A method according to claim 4, 5 or 6, characterised in that the silanising agent is applied during grinding.

8. A method according to claim 4, 5 or 6, characterised in that the ground surface is cleaned mechanically with a brush.

9. A method according to claim 8, characterised in that the silanising agent is simultaneously applied by means of the brush.

**Revendications**

1. Agent de silanisation contenant du $\gamma$-méthacryloyloxypropyltriméthoxysilane pour la réparation d'une prothèse dentaire en céramique avec une matière plastique, caractérisé en ce qu'il consiste en 50-95 % en volume de $\gamma$-méthacryloyloxypropyltriméthoxysilane et 5-50 % en volume de triméthyléthoxysilane.

2. Agent de silanisation selon la revendication 1, caractérisé en ce qu'il consiste en 85-95 % en volume de $\gamma$-méthacryloyloxypropyltriméthoxysilane et 5-15 % en volume de triméthyléthoxysilane.

3. Agent de silanisation selon la revendication 1 ou 2, caractérisé en ce qu'il consiste en 90 % en volume de $\gamma$-méthacryloyloxypropyltriméthoxysilane et 10 % en volume de triméthyléthoxysilane.

4. Procédé pour la silanisation d'une prothèse dentaire en céramique à réparer avec une matière plastique, caractérisé en ce que la prothèse dentaire en céramique est meulée à sec dans la région à réparer avec une meule fine et à une vitesse de rotation de 5 000 à 25 000 tr/min et un agent de silanisation consistant en 50-95 % en volume de $\gamma$-méthacryloyloxypropyltriméthoxysilane et 5-50 % en volume de triméthyléthoxysilane est appliqué sur la surface meulée.

5. Procédé selon la revendication 4, caractérisé en ce que l'on applique un agent de silanisation consistant en 85-95 % en volume de γ-méthacryloyloxypropyltriméthoxysilane et 5-15 % en volume de triméthyléthoxysilane.

6. Procédé selon la revendication 4 ou 5, caractérisé en ce que l'on applique un agent de silanisation consistant en 90 % en volume de γ-méthacryloyloxypropyltriméthoxysilane et 10 % en volume de triméthyléthoxysilane.

7. Procédé selon la revendication 4, 5 ou 6, caractérisé en ce que l'agent de silanisation est appliqué pendant le meulage.

8. Procédé selon la revendication 4, 5 ou 6, caractérisé en ce que la surface meulée est nettoyée mécaniquement avec une brosse.

9. Procédé selon la revendication 8, caractérisé en ce que l'agent de silanisation est appliqué simultanément avec la brosse.